# EUROPEAN PATENT APPLICATION

(11) **EP 2 357 224 A1**
(43) Date of publication of application: **17.08.2011**
(21) Application number: 09826325.4
(22) Date of filing: 22.09.2009
(51) Int. Cl.: C12N 7/00, C12N 15/34

(54) **METHOD FOR ISOLATING THE DNA OF THE AVIAN INFECTIOUS LARYNGOTRACHEITIS VIRUS FOR PCR ANALYSIS**

(30) Priority: 12.11.2008 KZ 20081238
(71) Applicant: "Repulican Governmental Enterprise on the right of Economic Management "Research Institute for Biological Safety Problems" of The Science, Zhambylskaya obl. 080409 (KZ)
(72) Inventor: SANDYBAYEV, Nurlan Tamambayevich, Zhambylskaya obl. 080409 (KZ); MAMADALIYEV, Seidigapbar Mamadaliyevich, Zhambylskaya obl. 080409 (KZ); ZHOLDYBAYEVA, Yelena Vital'yevna, Zhambylskaya obl. 080409 (KZ); SULTANKULOVA, Kulyaisan Turlybayevna, Zhambylskaya obl. 080409 (KZ); STROCHKOV, Vitaliy Mikhailovich, Zhambylskaya obl. 080409 (KZ); ZAITSEV, Valentin Luk'yanovich, Zhambylskaya obl. 080409 (KZ)
(74) Representative: Jeck, Anton
(86) International application number: PCT/KZ2009/000016
(87) International publication number: WO 2010/056093

(57) **Abstract**

The invention relates to the field of molecular biology, namely to methods of DNA extraction.

The gist of the method lies in ILT virus DNA extraction from virus-containing materials via lysis and protease inactivation, using the lysing buffer including guanidine isothiocyanate followed by DNA sorption on silicon dioxide particles.

The method set forth is a more efficient procedure, compared to the existing procedures for extraction of viral DNA for PCR, and has been developed for use in laboratories and research institutions engaged in PCR diagnosis of DNA-virus infections.

This method of DNA extraction is easy to use, time-saving, feasible in a practical way, and suitable for preparing specimens for amplification.

## Description

The invention relates to the field of molecular biology, namely to methods of extracting DNA.

In molecular biology and genetic engineering, a widely used phenolic method of DNA extraction employs proteinase K and 10% SDS for virus cleavage, protein withdrawal and DNA extraction. The disadvantages of this method are that it is laborious and not very effective and that it requires the use of such aggressive agents as chloroform and phenol (Poa-Chun Chang, Yuan-Ling Lee, Jui-Hung Shich, Happy K. Shich. Rapid differentiation of vaccine strains and field isolates of laryngotracheitis virus by restriction fragment length polymorphism of PCR products // J. Virol. Meth. 66 (1997) 179-186).

The method set forth herein is a more efficient procedure for extracting infectious laryngotracheitis (ILT) virus DNA from virus-containing materials for PCR assay and has been developed for use in laboratories and research institutions engaged in PCR diagnosis of DNA-virus infections.

The gist of the method for ILT virus DNA extraction from virus-containing materials lies in lysis and protease inactivation with guanidine isothiocyanate, followed by DNA sorption on silicon dioxide particles.

### Sorbent preparations

Silicon dioxide, SiO₂, (6 g) is suspended in deionized water (total volume 50 cm³) in a glass cylinder and balanced at room temperature for 24 h. Then the upper water phase is aspirated. Its volume varies depending on the original SiO₂. The residue is suspended in a shaker with subsequent addition of water (50 cm³), stirred, and left overnight at room temperature. Afterwards the upper water phase is aspirated; an equal volume of deionized water is added and left for 5 h. The upper phase is aspirated (becoming more transparent each time). The residue is stirred and the pH is adjusted to 2.0 with concentrated HCl. The prepared SiO₂ is poured into glass vessels in small aliquots and autoclaved at 121°C for 20 min. This solution is stored for 6 months at room temperature in the dark.

### Preparation of buffers

*Lysing buffer:* Guanidine isothiocyanate (12 g) is dissolved in 10 cm³ of 0.1 M Tris-HCl, pH 6.4, with 2.2 cm³ of 0.2 M EDTA, pH 8.0, and is supplemented with 0.26 g of Triton X-100.

*Note:* To prepare 0.1 M Tris-HCl, pH 6.4, pH of basic Tris (with molecular weight 121.14 g/mol) is adjusted with hydrochloric acid.

*Wash buffer:* Guanidine isothiocyanate (12 g) is dissolved in 10 cm³ of 0.1 M Tris-HCl, pH 6.4.

*Note:* Guanidine isothiocyanate is dissolved by heating in a water bath at 60-65°C.

***Warning:*** Heated guanidine isothiocyanate can emit toxic gas!!!

DNA is extracted from organ-tissue materials, from allantoic fluid and tracheal washes.
1. 900 Ol of the lysing buffer and 50 Ol of the previously prepared SiO₂ sorbent are stirred for 10 sec in a 1.5 cm³ tube, then the tested sample is added in the amount of 200 Ol; the mixture is stirred again for 10 sec and incubated at room temperature for 10-15 min.
2. The mixture is centrifuged for 1 min in a microcentrifuge ("MiniSpin", Eppendorf) at maximal speed. The supernatant is removed, 1 cm³ of the wash buffer is added and resuspended on a vortex, then centrifuged for 1 min at maximal speed.
3. The supernatant is removed and the procedure with the wash buffer (step 2) is repeated.
4. The supernatant is removed and 1 cm³ of 70% alcohol is added (the pellet is not resuspended!), the tubes are centrifuged at maximal speed for 1 min, and their content is washed again with 70% alcohol.
5. The supernatant is removed, and the sediment is dried in the open tubes at 56°C for 10 min.
6. The pellet is resuspended in 50 Ol of TE-buffer (10 mM of Tris-HCl, 1 mM of EDTA, pH 8.0), incubated at 56°C for 10 min, then centrifuged at maximal speed for 2 min.
7. The supernatant is transferred to another tube and is centrifuged again at maximal speed for 1 min to remove the remaining silica particles, then is transferred again to still another tube for subsequent use in PCR.

As a subject of the research into the "Otar" strain of the infectious laryngotracheitis virus, tracheal swabs were used as pathology material (trachea, larynx) from suspected cases. The results of the experiments confirm that the above described procedure allows extracting viral DNA suitable for PCR-assay in one hour, owing to rapid lysis of cells and DNA sorption on SiO₂ sorbent. The method is good because, compared to the phenolic method, there is no DNA loss; it is easy to use, time-saving, feasible in a practical way, and suitable for preparing specimens for amplification.

## Claims

1. method of extracting DNA of the infectious laryngotracheitis virus from virus-containing materials, which is distinguished by including guanidine isothiocyanate in the composition of the lysing buffer and by DNA sorption on silicon dioxide particles after lysis and protease inactivation.
